# EUROPEAN PATENT APPLICATION

(11) **EP 1 847 830 A1**
(43) Date of publication of application: **24.10.2007**
(21) Application number: 06008221.1
(22) Date of filing: 20.04.2006
(51) Int. Cl.: G01N 29/024, A61K 41/00, A23L 3/37

(54) **Determining optimal concentrations of protective additives for biomolecules**

(71) Applicant: TF Instruments, Inc., Monmouth Junction NJ 08852 (US)
(72) Inventor: Funck, Theodor, 37077 Göttingen (DE); Gau, daniel, 64404 Bickenbach (DE); Bierbaum, Hanna, 68163 Mannheim (DE)
(74) Representative: Katzameyer, Michael

(57) **Abstract**

The invention provides a method for determining optimal concentrations of additives which are capable to protect sensitive biomacromolecules, e.g. proteins or nucleic acids, against denaturation and aggregation in a solution comprising at least one species of a biomacromolecule and at least one species of a protective additive comprising:
a) treating said solution with ultrasonic waves; and
b) recording the value of one or more ultrasonic parameters, such as the ultrasonic velocity in said solution and/or the ultrasonic absorption of said solution.

The above method allows to detect critical changes of the ultrasonic parameter dependent from the concentration of the protective additive and indicative of interactions or changes of interactions between additive and biomacromolecules and/or between different biomacromolecules and/or changes of the conformation of the biomacromolecules. Thus, the determination of such changes can be used to determine an optimal concentration of said additive for protecting the biomacromolecule from denaturation and aggregation.

## Description

### Background

It is well known that certain low molecular substances can protect sensitive biomacromolecules against denaturation and irreversible aggregation. Such additives are e.g. polyhydroxy or polyether compounds. They protect the surface of the bio-molecule and prevent direct intermolecular contact which may lead to denaturation or irreversible aggregation. It is known that in the presence of such protective additives biomacromolecules may be stored in solution, may be stored frozen and re-thawed, or even be freeze-dried, stored over long periods of time and afterwards dissolved again by addition of aqueous media to produce solutions of undernatured biomacromolecules.

The determination of the effective concentration of such additives, which are required to ensure the protective effect is laborious and time consuming; solutions of the biomacromolecules have to be prepared with the addition of various concentrations of the respective protective substances. These samples have to be stored for longer time and then analyzed for denatured or aggregated biomacromolecules. Or they have to be frozen and stored for longer periods of time, re-thawed and analyzed for denatured or aggregated biomolecules. Or they have to be freeze-dried, stored for longer periods of time and then dissolved again by addition aqueous medium. Afterwards, the content of denatured or aggregated biomolecules must be determined. In any case, a statement on the protective effect of the additives is only possible after the long period of storage time.

Therefore, an object of the present invention is to provide an improved fast method for determining optimal concentrations of such a protective additive without storage and subsequent analysis.

Surprisingly, it has been found that a detection of the protective effect of such additives without storage and subsequent analysis is possible on the basis of highly sensitive and precise ultrasonic measurements. Measurements of the ultrasonic parameters in solutions of biomacromolecules containing increasing concentrations of protective additives using the sensitive ultrasonic resonator technology permit a reliable prediction of the protective effect in a sample. Characteristic changes of the ultrasonic parameters (such as ultrasonic velocity and ultrasonic absorption) with increasing concentration of the protective additive indicate reliably the critical concentration of the additive which is required for a protection of the biomacromolecule against denaturation and/or aggregation during storage, freeze-drying, storage and re-dissolution or freezing, storage and thawing. By applying such ultrasonic measurements the analysis of denaturation or aggregation after storage of the sample will no more be necessary.

Thus, the above-identified problem is solved according to the present invention by providing a method for determining optimal concentrations of such a protective additive in a solution based on ultrasonic measurements according to present claims 1-10.

### Description of the invention

The present invention is based on the finding that a detection of the protective effect of such additives without storage and subsequent analysis is possible on the basis of highly sensitive and precise ultrasonic measurements. Measurements of the ultrasonic parameters in solutions of biomacromolecules containing increasing concentrations of protective additives using the sensitive ultrasonic resonator technology permit a reliable prediction of the protective effect in a sample. Characteristic changes of the ultrasonic parameters (such as ultrasonic velocity and ultrasonic absorption) with increasing concentration of the protective additive indicate reliably the optimal concentration of the additive which is appropriate for a protection of the biomacromolecule, e.g. during freezing, freeze-drying, storage and redissolution or freezing, storage and thawing.

The method of the present invention for determining optimal concentrations of additives which are capable to protect sensitive biomacromolecules against denaturation and aggregation in a solution comprising at least one species of a biomacromolecule which is sensitive to denaturation and aggregation and at least one species of a protective additive comprises the following steps:
a) treating a solution comprising at least one species of a biomacromolecule which is sensitive to denaturation and aggregation and at least one species of a protective additive with ultrasonic waves; and
b) recording the value of one or more ultrasonic parameters, such as the ultrasonic velocity in said solution and/or the ultrasonic absorption of said solution.

Typically, the method also comprises the step of comparing the values of said ultrasonic parameter obtained in step b) with reference values. Said reference values may be the values of the same parameter recorded in a reference solution having the same concentration of the same protective additive but which lacks the biomacromolecule.

In a specific embodiment of the method of the present invention, a series of solutions with increasing concentrations of a protective additive is treated with ultrasonic waves and the values obtained in step b) or the difference between the values obtained in step b) and reference values are plotted against the concentration of the protective additive.

The method of the present invention allows to detect critical changes of the ultrasonic parameter, which changes are dependent from the concentration of the protective additive and indicative of interactions or (critical) changes of interactions between additive and biomacromolecules and/or between different biomacromolecules and/or changes of the conformation of the biomacromolecules. Thus, the determination of such changes can be used to determine an optimal concentration of said additive for protecting the biomacromolecule from denaturation and aggregation.

Suitable biomacromolecules are any macromolecules which are sensitive to denaturation and aggregation. Specific, but not limiting examples thereof are biological macromolecules such as peptides, proteins, nucleic acids and natural or synthetic derivatives thereof. Further suitable biomacromolecules will be evident for the skilled artisan.

Suitable additives are any additives which are capable to protect biomacromolecules from denaturation and aggregation. The term "proctecting" as used herein includes "preventing" as well as "inhibiting". A great variety of such additives are well known in the art and available for the skilled artisan. Specific, but not limiting examples of suitable additives are oligo- or polyhydroxy compounds and oligo- or polyether compounds. In a more specific embodiment, the additive is a sugar, in particular glucose, saccharose or trehalose. Evidently, a mixture of different additives may also be used.

In a preferred embodiment of the method of the present invention, the ultrasonic parameter is the ultrasonic velocity in a solution containing the additive and changes of this velocity with increasing concentration of the additive(s) are recorded.

Preferably, the ultrasonic measurements are effected using an ultrasonic resonator. Suitable ultrasonic resonators are known in the art and, e.g., commercially available from TF Instruments Inc., NJ, USA, or TF Instruments GmbH, Heidelberg (e.g. the ResoScan System).

## Claims

1. A method for determining optimal concentrations of additives which are capable to protect sensitive biomacromolecules against denaturation and aggregation in a solution comprising at least one species of a biomacromolecule which is sensitive to denaturation and aggregation and at least one species of a protective additive, which method comprises:
a) treating a solution comprising at least one species of a biomacromolecule which is sensitive to denaturation and aggregation and at least one species of a protective additive with ultrasonic waves;
b) recording the value of at least one ultrasonic parameter selected from the ultrasonic velocity in said solution and the ultrasonic absorption of said solution.

2. The method according to claim 1, further comprising
c) comparing the values of said ultrasonic parameter obtained in step b) with reference values.

3. The method according to claim 1 or 2, wherein the biomacromolecules are selected from the group consisting of peptides, proteins, nucleic acids and natural or synthetic derivatives thereof.

4. The method according to any one of claims 1-3, wherein the additives are selected from the group consisting of oligo- or polyhydroxy compounds and oligo- or polyether compounds.

5. The method according to claim 4, wherein the additive is a sugar, in particular glucose, saccharose or trehalose.

6. The method according to any one of claims 1-5, wherein the values obtained in step b) are compared with the values of the same parameter recorded in a reference solution having the same concentration of the same protective additive but which lacks the biomacromolecule.

7. The method according to any one of claims 1-6, wherein a series of solutions with increasing concentrations of a protective additive is treated with ultrasonic waves and the values obtained in step b) or the difference between the values obtained in step b) and reference values are plotted against the concentration of the protective additive.

8. The method according to any one of claims 1-7, wherein a critical change of the ultrasonic parameter which is dependent from the concentration of the protective additive is observed and used to determine an optimal concentration of said additive for protecting the biomacromolecule from denaturation and aggregation.

9. The method according to any one of claims 1-8, wherein said ultrasonic parameter is the ultrasonic velocity in said solution.

10. The method according to any one of claims 1-9, wherein an ultrasonic resonator is used.
